# EUROPEAN PATENT APPLICATION

(11) **EP 4 541 270 A1**
(43) Date of publication of application: **23.04.2025**
(21) Application number: 23839979.4
(22) Date of filing: 13.07.2023
(51) Int. Cl.: A61B 5/024, A61B 5/00, A61B 5/0245, G16H 50/20

(54) **METHOD, PROGRAM, AND DEVICE FOR GENERATING LONG-TERM HEART RATE VARIABILITY ON BASIS OF SHORT-TERM MEASUREMENT SIGNALS**

(30) Priority: 14.07.2022 KR 20220086817; 12.07.2023 KR 20230090213
(71) Applicant: Medical AI Co., Ltd., Seoul 06180 (KR)
(72) Inventor: KWON, Joonmyoung, Seoul 06180 (KR); KANG, Seonmi, Seoul 06180 (KR); LIM, Seonyu, Seoul 06180 (KR)
(74) Representative: Patentanwaltskanzlei Matschnig & Forsthuber OG
(86) International application number: PCT/KR2023/009991
(87) International publication number: WO 2024/014888

(57) **Abstract**

According to one embodiment of the present, there is disclosed a method, program, and device for generating long-term heart rate variability based on a short-term measured heart signal, which are performed by a computing device. The method may include: obtaining heart data including a heart signal measured within a first reference time; and generating heart rate variability for a second reference time, which is a value larger than the first reference time, based on the heart data by using a pre-trained deep learning model.

## Description

### Technical Field

The present disclosure relates to artificial intelligence technology in the medical field, and more particularly, to a method capable of generating heart rate variability itself, which is similar to that extracted from a long-term measured heart signal, from a short-term measured heart signal by utilizing artificial intelligence and also capable of extracting a feature from the generated heart rate variability.

### Background Art

The heart does not always beat at a constant rate. The interval between heartbeats increases or decreases. In a healthy state, the variation in the interval between heartbeats is generally large and complex. In contrast, in a state involving disease or stress, the complexity of the interval between heartbeats is generally significantly reduced. A large variation in heartbeats indicates excellent adaptability to changes inside or outside the body, while a small variation in heartbeats indicates decreased adaptability to changes inside the body or in an external environment.

As described above, it is possible to know the state of our body through aspects of the variation of heartbeats, and the concept derived from this is heart rate variability (HRV) . Heart rate variability is the numerical representation of the aspect of the variation when the intervals between heartbeats are measured sequentially. In other words, heart rate variability is a collection of values obtained by measuring the intervals between heartbeats, such as the interval between first and second beats, the interval between second and third beats, etc., and representing the aspect of the intervals in numbers.

Heart rate variability usually uses a recording of 24-hour analysis or 5-minute analysis as the reference of the gold standard. Several papers have studied the suitability of using ultra-short-term (<5 minutes) heart rate variability, and have shown that as the time decreases, the heart rate variability deviates more from the numeral value of the gold standard. In particular, there were considerable differences in the features of heart rate variability related to frequency. Therefore, it is generally recommended that a heart signal is measured for at least 5 minutes to analyze heart rate variability.

However, from a user's perspective, it is not easy to maintain a state of continuous concentration to measure a heart signal for 5 minutes. Accordingly, noise attributable to movement may easily be added during the process of measuring a heart signal for 5 minutes. Especially in 24-hour measurement, a large amount of noise is bound to be included. Such noise reduces the reliability of results in the analysis of heart rate variability. Therefore, if performance up to that of the gold standard can be achieved with short-term measurement, it will be considerably helpful in terms of user convenience, and the reliability of the analysis of heart rate variability can be increased by limiting noise.

### Disclosure

### Technical Problem

An object of the present disclosure is to provide a method capable of generating heart rate variability itself, which is similar to that extracted from a long-term measured heart signal, from a short-term measured heart signal by utilizing artificial intelligence and also capable of extracting a feature from the generated heart rate variability.

However, the objects to be accomplished by the present disclosure are not limited to the object mentioned above, and other objects not mentioned may be clearly understood based on the following description.

### Technical Solution

According to one embodiment of the present disclosure for achieving the above-described object, there is disclosed a method of generating long-term heart rate variability based on a short-term measured heart signal. The method may include: obtaining heart data including a heart signal measured within a first reference time; and generating heart rate variability for a second reference time, which is a value larger than the first reference time, based on the heart data by using a pre-trained deep learning model.

Alternatively, the heart data may further include at least one of the biological information, disease information, or physical activity information of a person from whom the heart signal was measured.

Alternatively, generating the heart rate variability for the second reference time, which is the value larger than the first reference time, based on the heart data by using the pre-trained deep learning model may include: computing a mean and a standard deviation for the heart rate variability for the second reference time by inputting the heart data to a pre-trained first model; predicting a probability distribution for the heart rate variability for the second reference time based on the mean and the standard deviation; and computing values included in the heart rate variability for the second reference time by performing sampling based on the predicted probability distribution.

Alternatively, computing the values included in the heart rate variability for the second reference time by performing the sampling based on the predicted probability distribution may include: when the predicted probability distribution corresponds to a normal distribution, computing the values included in the heart rate variability for the second reference time by performing random sampling on the predicted probability distribution.

Alternatively, computing the values included in the heart rate variability for the second reference time by performing the sampling based on the predicted probability distribution includes: when the predicted probability distribution does not correspond to a normal distribution, performing modeling on the predicted probability distribution; and computing the values included in the heart rate variability for the second reference time by performing random sampling on a probability distribution generated through the modeling.

Alternatively, computing the values included in the heart rate variability for the second reference time by performing the sampling based on the predicted probability distribution may include: when the predicted probability distribution corresponds to a normal distribution, computing the values included in the heart rate variability for the second reference time by performing random sampling on the predicted probability distribution.

Alternatively, computing the values included in the heart rate variability for the second reference time by performing the sampling based on the predicted probability distribution includes: when the predicted probability distribution does not correspond to a normal distribution, performing modeling on the predicted probability distribution; and computing the values included in the heart rate variability for the second reference time by performing random sampling on a probability distribution generated through the modeling.

Alternatively, the modeling performed on the predicted probability distribution may include a Gaussian mixture model or a normalizing flow.

Alternatively, computing the values included in the heart rate variability for the second reference time by performing the sampling based on the predicted probability distribution includes: determining whether the predicted probability distribution corresponds to a normal distribution; selecting one of a plurality of sampling techniques for extracting the heart rate variability for the second reference time based on a result of the determination; and computing values included in the heart rate variability for the second reference time by performing sampling on the probability distribution via the selected sampling technique.

Alternatively, generating the heart rate variability for the second reference time, which is the value larger than the first reference time, based on the heart data by using the pre-trained deep learning model may further include: rearranging the order of the values included in the generated heart rate variability for the second reference time by using a pre-trained second model based on self-attention.

Alternatively, rearranging the order of the values included in the generated heart rate variability for the second reference time by using the pre-trained second model based on self-attention includes: generating an attention map indicating the correlations between the values included in the heart rate variability by inputting the heart rate variability for the second reference time to the second model; and rearranging the order of the values included in the heart rate variability based on the attention map.

Alternatively, generating the heart rate variability for the second reference time, which is the value larger than the first reference time, based on the heart data by using the pre-trained deep learning model may include: computing the values included in the heart rate variability for the second reference time by inputting the heart data to a third model, which is a pre-trained generative model.

Alternatively, generating the heart rate variability for the second reference time, which is the value larger than the first reference time, based on the heart data by using the pre-trained deep learning model may include: computing the values included in the heart rate variability for the second reference time by inputting the heart data to a fourth model, which is a pre-trained sequence-to-sequence model.

According to one embodiment of the present disclosure for achieving the above-described object, there is disclosed a computer program stored in a computer-readable storage medium. The computer program performs operations for generating long-term heart rate variability based on a short-term measured heart signal when executed on one or more processors. In this case, the operations may include operations of: obtaining heart data including a heart signal measured within a first reference time; and generating heart rate variability for a second reference time, which is a value larger than the first reference time, based on the heart data by using a pre-trained deep learning model.

According to one embodiment of the present disclosure for achieving the above-described object, there is disclosed a computing device for generating long-term heart rate variability based on a short-term measured heart signal. The computing device may include: a processor including at least one core; memory including program codes executable on the processor; and a network unit configured to obtain heart data including a heart signal measured within a first reference time. In this case, the processor may generate heart rate variability for a second reference time, which is a value larger than the first reference time, based on the heart data by using a pre-trained deep learning model.

### Advantageous Effects

According to the present disclosure, in a situation where a long-term heart signal needs to be measured, the performance of long-term measurement may be achieved with only short-term measurement, so that user convenience can be significantly increased.

Furthermore, the heart rate variability itself may be generated with only short-term measurement, so that the probability that noise occurs during the measurement of the heart signal can be significantly reduced.

Moreover, the heart rate variability itself, which is the same as that extracted from a long-term measured heart signal, may be generated from a short-term measured heart signal, so that a system having general usability that can be directly applied to various diagnostic algorithms utilizing the heart rate variability can be implemented.

### Description of Drawings

FIG. 1 is a block diagram of a computing device according to one embodiment of the present disclosure;
FIG. 2 is a conceptual diagram showing a heart signal measured within a first reference time and heart rate variability for a second reference time according to one embodiment of the present disclosure;
FIG. 3 is a block diagram showing a process of generating heart rate variability for a second reference time based on the heart data measured within a first reference time according to one embodiment of the present disclosure;
FIG. 4 is a block diagram showing a process of generating heart rate variability for a second reference time by using a deep learning model according to one embodiment of the present disclosure;
FIG. 5 is a block diagram showing a process of extracting a heart rate variability feature for a second reference time by using a deep learning model according to one embodiment of the present disclosure; and
FIG. 6 is a flowchart showing a method of generating long-term heart rate variability based on a short-term measured heart signal according to one embodiment of the present disclosure.

### Mode for Invention

Embodiments of the present disclosure will be described in detail below with reference to the accompanying drawings so that those having ordinary skill in the art of the present disclosure (hereinafter referred to as those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

The term "obtaining" used herein can be understood to mean not only receiving data over a wired/wireless communication network connecting with an external device or a system, but also generating data in an on-device form.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "model" used herein may be understood as a system implemented using mathematical concepts and language to solve a specific problem, a set of software units intended to solve a specific problem, or an abstract model for a process intended to solve a specific problem. For example, a neural network "model" may refer to an overall system implemented as a neural network that is provided with problem-solving capabilities through training. In this case, the neural network may be provided with problem-solving capabilities by optimizing parameters connecting nodes or neurons through training. The neural network "model" may include a single neural network, or may include a neural network set in which multiple neural networks are combined together.

The foregoing descriptions of the terms are intended to help to understand the present disclosure. Accordingly, it should be noted that unless the above-described terms are explicitly described as limiting the content of the present disclosure, the terms in the content of the present disclosure are not used in the sense of limiting the technical spirit of the present disclosure.

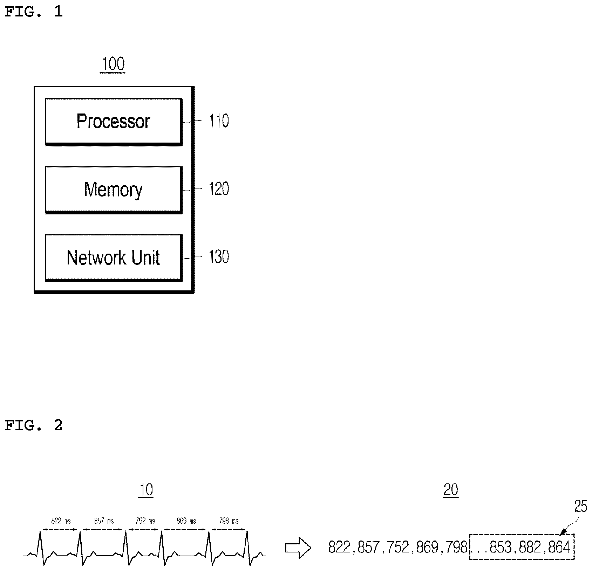

FIG. 1 is a block diagram of a computing device according to one embodiment of the present disclosure.

The computing device 100 according to the one embodiment of the present disclosure may be a hardware device or a part of a hardware device that performs the comprehensive processing and computation of data, or may be a software-based computing environment that is connected to a communication network. For example, the computing device 100 may be a server that is a principal agent performing an intensive data processing function and sharing resources, or may be a client that shares resources through interaction with a server or a specific terminal. Furthermore, the computing device 100 may be a cloud system in which pluralities of servers and clients comprehensively process data while interacting with each other. Furthermore, the computing device 100 may be a component of a medical robot. Since the above-described description is only one example related to the type of the computing device 100, the type of the computing device 100 may be configured in various manners within a range understandable to those skilled in the art based on the content of the present disclosure.

Referring to FIG. 1, the computing device 100 according to the one embodiment of the present disclosure may include a processor 110, memory 120, and a network unit 130. However, FIG. 1 shows only an example, and the computing device 100 may include other components for implementing a computing environment. Alternatively, only some of the components disclosed above may be included in the computing device 100.

The processor 110 according to one embodiment of the present disclosure may be understood as a constituent unit including hardware and/or software for performing computing operation. For example, the processor 110 may read a computer program and perform data processing for machine learning. The processor 110 may process computational processes such as the processing of input data for machine learning, the extraction of features for machine learning, and the calculation of errors based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA). Since the types of processor 110 described above are only examples, the type of processor 110 may be configured in various manners within a range understandable to those skilled in the art based on the content of the present disclosure.

The processor 110 may generate heart rate variability for a second reference time, which is a value larger than a first reference time, based on heart data including a heart signal measured within the first reference time by using a pre-trained deep learning model. In this case, the heart signal may be understood as a signal indicating the activity or state of the heart. Furthermore, the reference time may be a value preset by the user by taking into consideration various factors such as the measurement environment of the heart signal, the purpose of use, and/or the like. For example, the heart signal may include a 12-lead electrocardiogram, a Holter electrocardiogram, or a photoplethysmography (PPG). The first reference time may be 10 to 30 seconds, and the second reference time may be 5 to 24 hours in accordance with the gold standard for the analysis of heart rate variability.

The processor 110 may compute the features of the heart rate variability from the heart rate variability for the second reference time generated through the deep learning model. For example, the processor 110 may compute the features of the heart rate variability, such as the standard deviation of RR interval (SDRR), mean RR, the standard deviation of all NN intervals (SDNN), the root mean square of the successive differences (RMSSD), power in low frequency range (LF), or power in high frequency range (HF), through the mathematical operations of the values included in the heart rate variability for the second reference time. Furthermore, the processor 110 may compute a value required for diagnosis, such as stress index, stress resistance, autonomic nervous system balance, autonomic nervous system activity, fatigue, depression level, or anxiety disorder level, by combining the computed features of the heart rate variability.

The memory 120 according to one embodiment of the present disclosure may be understood as a constituent unit including hardware and/or software for storing and managing data that is processed in the computing device 100. That is, the memory 120 may store any type of data generated or determined by the processor 110 and any type of data received by the network unit 130. For example, the memory 120 may include at least one type of storage medium of a flash memory type, hard disk type, multimedia card micro type, and card type memory, random access memory (RAM), static random access memory (SRAM), read-only memory (ROM), electrically erasable programmable read-only memory (EEPROM), programmable read-only memory (PROM), magnetic memory, a magnetic disk, and an optical disk. Furthermore, the memory 120 may include a database system that controls and manages data in a predetermined system. Since the types of memory 120 described above are only examples, the type of memory 120 may be configured in various manners within a range understandable to those skilled in the art based on the content of the present disclosure.

The memory 120 may structure, organize, and manage the data required for the processor 110 to perform computation, the combination of data, and program codes executable on the processor 110. For example, the memory 120 may store the heart data received through the network unit 130 to be described later. The memory 120 may store program codes adapted to operate the neural network model to receive heart data and perform learning, program codes adapted to operate the neural network model to receive heart data and perform inference in accordance with the purpose of use of the computing device 100, processed data generated as program codes are executed, and/or the like.

The network unit 130 according to one embodiment of the present disclosure may be understood as a constituent unit that transmits and receives data through any type of known wired/wireless communication system. For example, the network unit 130 may perform data transmission and reception using a wired/wireless communication system such as a local area network (LAN), a wideband code division multiple access (WCDMA) network, a long term evolution (LTE) network, the wireless broadband Internet (WiBro), a 5th generation mobile communication (5G) network, a ultra wide-band wireless communication network, a ZigBee network, a radio frequency (RF) communication network, a wireless LAN, a wireless fidelity network, a near field communication (NFC) network, or a Bluetooth network. Since the above-described communication systems are only examples, the wired/wireless communication system for the data transmission and reception of the network unit 130 may be applied in various manners other than the above-described examples.

The network unit 130 may receive the data required for the processor 110 to perform computation through wired/wireless communication with any system or client or the like. Furthermore, the network unit 130 may transmit the data generated through the computation of the processor 110 through wired/wireless communication with any system or client or the like. For example, the network unit 130 may receive medical data through communication with a database within a hospital environment, a cloud server configured to perform tasks such as the standardization of medical data, a computing device, or the like. The network unit 130 may transmit the output data of the neural network model, intermediate data and processed data acquired from the computation process of the processor 110, etc. through communication with the above-described database, server, or computing device.

FIG. 2 is a conceptual diagram showing a heart signal measured within a first reference time and heart rate variability for a second reference time according to one embodiment of the present disclosure.

The numerical values representing peak-to-peak intervals in the heart signal 10 measured within the first reference time represent the values included in the heart rate variability within the first reference time. For example, as shown in FIG. 2, the numerical value "822" in the first peak-to-peak interval "822 ms" represents the first value of the heart rate variability, and the numerical value "857" in the second peak-to-peak interval "857 ms" represents the second value of the heart rate variability. When the numerical values representing the peak-to-peak intervals during the time for which the heart signal 10 is measured within the first reference time are listed in this manner, the heart rate variability within the first reference time may be calculated.

Referring to FIG. 2, the heart rate variability 20 for the second reference time generated from the heart signal 10 measured within the first reference time by the computing device 100 according to one embodiment of the present disclosure may include the values, included in the heart rate variability within the first reference time, and newly generated values 25. The newly generated values 25 may be understood as values that are computed based on the results of predicting what characteristics and aspects the heart signal 10 measured within the first reference time would exhibit if measured up to the second reference time. That is, the computing device 100 may generate heart rate variability values predicted to be within a period equal to or longer than the first reference time and equal to or shorter than the second reference time by inputting the heart signal 10 measured within the first reference time to the deep learning model. Furthermore, the computing device 100 may generate the heart rate variability for the second reference time by connecting the heart rate variability values, computable from the heart signal 10 measured within the first reference time, and the newly generated values.

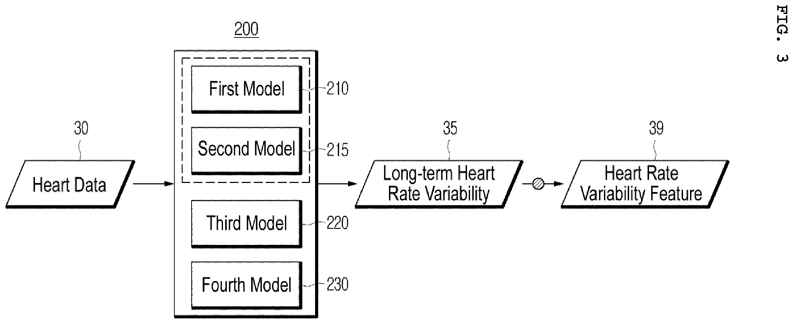

FIG. 3 is a block diagram showing a process of generating heart rate variability for a second reference time based on the heart data measured within a first reference time according to one embodiment of the present disclosure.

Referring to FIG. 3, heart data 30 according to one embodiment of the present disclosure may include a heart signal, such as an electrocardiogram, measured within the first reference time. The heart data 30 may further include at least one of the biological information, disease information, and physical activity information of a person from whom the heart signal was measured. The biological information may include the age, gender, height, weight, and/or the like of the person from whom the heart signal was measured. The disease information may include the presence or absence of a disease upon measurement and disease history. The physical activity information may include a condition and an activity state upon measurement.

The computing device 100 according to one embodiment of the present disclosure may compute long-term heart rate variability 35 corresponding to the heart rate variability for the second reference time by inputting the heart data 30 to a deep learning model 200. The deep learning model 200 may include a first model 210 configured to predict a probability distribution of heart rate variability and sample heart rate variability from the predicted distribution, a second model 215 configured to refine the heart rate variability sampled through the first model 210, a third model 220 corresponding to a generative model, and a fourth model 230 corresponding to a sequence-to-sequence model. The computing device 100 may compute the long-term heart rate variability 35 by selectively or complexly using the first model 210 to the fourth model 230 according to the type of heart signal included in the heart data 30, a user's intention, and/or the like.

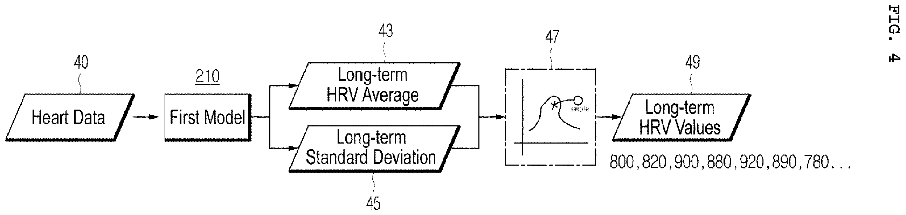

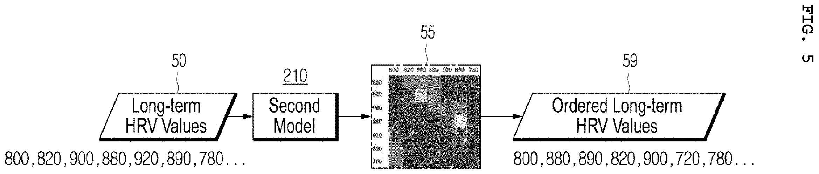

For reference, a process of generating the long-term heart rate variability 35 using the first model 210 and the second model 215 will be specifically described later with reference to FIGS. 4 and 5.

The third model 220 may learn the distribution of heart rate variability within the first reference time from the heart data 30, and may generate the long-term heart rate variability 35 corresponding to the heart rate variability for the second reference time from the learned distribution. That is, the third model 220 may newly generate the long-term heart rate variability 35, which is heart rate variability similar to that generated from the signal actually measured up to the second reference time, by determining the distribution of the heart rate variability computed from the heart data 30 including the signal actually measured within the first reference time. For example, the third model 220 may include a generative neural network such as a generative adversarial network (GAN) or a variational autoencoder (VAE). However, the types of neural network of the third model 220 described above are only examples, and thus, various generative neural networks other than the above-described examples may be applied as the neural network of the third model 220.

The fourth model 230 may learn the features of the heart data 30 changing over time, and may generate the long-term heart rate variability 35 corresponding to the heart rate variability for the second reference time based on the results of the learning. That is, the fourth model 230 may generate the long-term heart rate variability 35 for the second reference time by predicting how the heart data 30 will change after the first reference time. For example, the fourth model 230 may include a sequence-to-sequence model such as a recurrent neural network (RNN), a long short-term memory (LSTM), or the like. However, the types of neural network of the fourth model 230 described above are only examples, and thus, various generative neural networks other than the examples described above may be applied as the neural network of the fourth model 230.

The computing device 100 may compute a heart rate variability feature 39 from the long-term heart rate variability 35 corresponding to the heart rate variability for the second reference time. In this case, the heart rate variability feature 39 may include SDRR, mean RR, SDNN, RMSSD, LF, HF, and/or the like. The computing device 100 generates the long-term heart rate variability 35 compliant with the gold standard and then extracts the heart rate variability feature 39 from the long-term heart rate variability 35, rather than directly extracting the heart rate variability feature 39 from the heart data 30 not compliant with the gold standard, so that various features can be easily extracted.

FIG. 4 is a block diagram showing a process of generating heart rate variability for a second reference time by using a deep learning model according to one embodiment of the present disclosure. Furthermore, FIG. 5 is a block diagram showing a process of extracting a heart rate variability feature for a second reference time by using a deep learning model according to one embodiment of the present disclosure.

Referring to FIG. 4, the computing device 100 may compute a mean 43 and a standard deviation 45 for long-term heart rate variability corresponding to the heart rate variability for the second reference time by inputting heart data 40, including a heart signal measured within a first reference time, to a first model 210. Although the first model 210 may be a model trained based on supervised learning in this case, the first model 210 may be trained based on a learning method applicable to the content disclosed in the present disclosure, other than the supervised learning.

The computing device 100 may predict the probability distribution 47 of the long-term heart rate variability by using the mean 43 and the standard deviation 45 for the long-term heart rate variability. Then, the computing device 100 may sample long-term heart rate variability values 49 from the predicted probability distribution 47. In this case, the sampling may be understood as a process of extracting desired values from a probability distribution. For example, when the predicted probability distribution 47 corresponds to a normal distribution, the computing device 100 may compute the long-term heart rate variability values 49 by performing random sampling on the predicted probability distribution 47. In other words, when the predicted probability distribution 47 corresponds to a normal distribution, the computing device 100 may generate the long-term heart rate variability values 49 by randomly extracting values present in the predicted probability distribution 47 until the second reference time is satisfied. In contrast, when the predicted probability distribution 47 does not correspond to a normal distribution, the computing device 100 may perform modeling on the predicted probability distribution 47. In this case, the modeling performed on the probability distribution 47 is a task of transforming the probability distribution 47, and may include modeling such as a Gaussian mixture model or a normalizing flow. In other words, even when the predicted probability distribution 47 does not follow a normal distribution, the computing device 100 may perform a task of processing the probability distribution 47 so that it is easy to extract desired values from the probability distribution 47. Furthermore, the computing device 100 may compute the long-term heart rate variability values 49 by performing random sampling on the modeled probability distribution.

Meanwhile, the computing device 100 may use other sampling techniques in addition to the above-described random sampling. For example, first, the computing device 100 may determine whether the predicted probability distribution 47 corresponds to a normal distribution. When the predicted probability distribution 47 corresponds to a normal distribution, the computing device 100 may perform random sampling to extract samples present in the normal distribution and generate the long-term heart rate variability values 49. When the predicted probability distribution 47 does not correspond to a normal distribution, the probability that an error will occur in the results of performing random sampling increases, so that the computing device 100 may select another sampling technique, such as a clustering technique, based on the distance of samples to perform sampling on the predicted probability distribution 47 and extract the long-term heart rate variability values 49. That is, the computing device 100 may select one of the plurality of sampling techniques depending on whether the predicted probability distribution 47 corresponds to a normal distribution and then perform sampling.

Referring to FIG. 5, the computing device 100 may rearrange the order of long-term heart rate variability values 50 by using the second model 220 based on self-attention. Since the long-term heart rate variability values 50 are generated by sampling the probability distribution predicted through the first model 210 as shown in FIG. 4, they do not have the order information of the original heart data input to the first model 210. This is not a significant problem when heart rate variability features (e.g., SDNN, etc.) for which order information is not important are computed, but it may be a problem when heart rate variability features (e.g., RMSSD, etc.) for which order information is important are computed. Accordingly, the computing device 100 may rearrange the order of the long-term heart rate variability values 50 by inputting the long-term heart rate variability values 50 to the second model 220 based on self-attention so that order information can be added to the long-term heart rate variability values 50 generated by sampling the probability distribution predicted through the first model 210.

More specifically, the computing device 100 may generate an attention map 55 representing the correlations between the long-term heart rate variability values 50 by inputting the long-term heart rate variability values 50 to the second model. The attention map may represent how highly correlated other long-term heart rate variability values are based on a specific long-term heart rate variability value. In this case, a high correlation may mean being close in order. For example, assuming that the value most highly correlated with the long-term heart rate variability value "800" corresponding to a first value is 890, the area where 800 and 890 are matched with each other in an attention map may be illustrated in the darkest color. Through this illustration on the attention map, it may be interpreted that it is desirable for 890 to be disposed after 800 in terms of long-term heart rate variability. That is, the computing device 100 may determine the order of the long-term heart rate variability values 50 through the attention map and rearrange them, and may generate the long-term heart rate variability values 59 arranged in order.

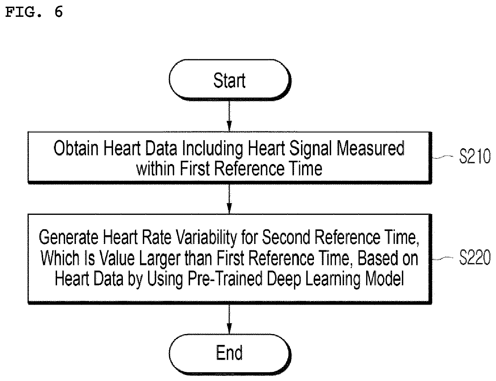

FIG. 6 is a flowchart showing a method of generating long-term heart rate variability based on a short-term measured heart signal according to one embodiment of the present disclosure.

Referring to FIG. 6, the computing device 100 according to one embodiment of the present disclosure may obtain heart data including a heart signal measured within a first reference time in step S210. For example, when the computing device 100 does not have a separate measurement unit, the computing device 100 may obtain heart data including a heart signal, measured within the first reference time, through wired or wireless communication with equipment for measuring heart signals. When the computing device 100 has a separate measurement unit, the computing device 100 may generate heart data by measuring a heart signal within the first reference time via the measurement unit. Meanwhile, the heart data may further include at least one of the biological information, disease information, or physical activity information of a person stored when the heart signal was measured, together with the heart signal measured within the first reference time.

The computing device 100 may generate heart rate variability for a second reference time, which is a value larger than that of the first reference time, based on the heart data by using a pre-trained deep learning model in step S220. More specifically, the computing device 100 may compute an average and a standard deviation for the heart rate variability for the second reference time by inputting the heart data to the pre-trained first model. The computing device 100 may predict the probability distribution of the heart rate variability for the second reference time based on the average and the standard deviation. The computing device 100 may compute the values included in the heart rate variability for the second reference time by performing sampling based on the predicted probability distribution. Furthermore, the computing device 100 may rearrange the order of the computed values included in the heart rate variability for the second reference time by using the pre-trained second model based on self-attention. Furthermore, the computing device 100 may compute the values included in the heart rate variability of the second reference time by inputting the heart data to the third model, which is a pre-trained generative model. Alternatively, the computing device 100 may compute the values included in the heart rate variability for the second reference time by inputting the heart data to the fourth model, which is a pre-trained sequence-to-sequence model.

Although not disclosed in FIG. 6, the computing device 100 may extract the features of the heart rate variability for the second reference time based on the values included in the heart rate variability for the second reference time computed through step S210.

The various embodiments of the present disclosure described above may be combined with one or more additional embodiments, and may be changed within the range understandable to those skilled in the art in light of the above detailed description. The embodiments of the present disclosure should be understood as illustrative but not restrictive in all respects. For example, individual components described as unitary may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

## Claims

1. A method of generating long-term heart rate variability based on a short-term measured heart signal, the method being performed by a computing device including at least one processor, the method comprising:
obtaining heart data including a heart signal measured within a first reference time; and
generating heart rate variability for a second reference time, which is a value larger than the first reference time, based on the heart data by using a pre-trained deep learning model.

2. The method of claim 1, wherein the heart data further includes at least one of biological information, disease information, or physical activity information of a person from whom the heart signal was measured.

3. The method of claim 1, wherein generating the heart rate variability for the second reference time, which is the value larger than the first reference time, based on the heart data by using the pre-trained deep learning model comprises:
computing a mean and a standard deviation for the heart rate variability for the second reference time by inputting the heart data to a pre-trained first model;
predicting a probability distribution for the heart rate variability for the second reference time based on the mean and the standard deviation; and
computing values included in the heart rate variability for the second reference time by performing sampling based on the predicted probability distribution.

4. The method of claim 3, wherein computing the values included in the heart rate variability for the second reference time by performing the sampling based on the predicted probability distribution comprises:
when the predicted probability distribution corresponds to a normal distribution,
computing the values included in the heart rate variability for the second reference time by performing random sampling on the predicted probability distribution.

5. The method of claim 3, wherein computing the values included in the heart rate variability for the second reference time by performing the sampling based on the predicted probability distribution comprises:
when the predicted probability distribution does not correspond to a normal distribution,
performing modeling on the predicted probability distribution; and
computing the values included in the heart rate variability for the second reference time by performing random sampling on a probability distribution generated through the modeling.

6. The method of claim 5, wherein the modeling performed on the predicted probability distribution includes a Gaussian mixture model or a normalizing flow.

7. The method of claim 3, wherein computing the values included in the heart rate variability for the second reference time by performing the sampling based on the predicted probability distribution comprises:
determining whether the predicted probability distribution corresponds to a normal distribution;
selecting one of a plurality of sampling techniques for extracting the heart rate variability for the second reference time based on a result of the determination; and
computing values included in the heart rate variability for the second reference time by performing sampling on the probability distribution via the selected sampling technique.

8. The method of claim 3, wherein generating the heart rate variability for the second reference time, which is the value larger than the first reference time, based on the heart data by using the pre-trained deep learning model further comprises:
rearranging an order of the values included in the generated heart rate variability for the second reference time by using a pre-trained second model based on self-attention.

9. The method of claim 8, wherein rearranging the order of the values included in the generated heart rate variability for the second reference time by using the pre-trained second model based on self-attention comprises:
generating an attention map indicating correlations between the values included in the heart rate variability by inputting the heart rate variability for the second reference time to the second model; and
rearranging the order of the values included in the heart rate variability based on the attention map.

10. The method of claim 1, wherein generating the heart rate variability for the second reference time, which is the value larger than the first reference time, based on the heart data by using the pre-trained deep learning model comprises:
computing the values included in the heart rate variability for the second reference time by inputting the heart data to a third model, which is a pre-trained generative model.

11. The method of claim 1, wherein generating the heart rate variability for the second reference time, which is the value larger than the first reference time, based on the heart data by using the pre-trained deep learning model comprises:
computing the values included in the heart rate variability for the second reference time by inputting the heart data to a fourth model, which is a pre-trained sequence-to-sequence model.

12. A computer program stored in a computer-readable storage medium, the computer program performing operations for generating long-term heart rate variability based on a short-term measured heart signal when executed on one or more processors, wherein the operations comprise operations of:
obtaining heart data including a heart signal measured within a first reference time; and
generating heart rate variability for a second reference time, which is a value larger than the first reference time, based on the heart data by using a pre-trained deep learning model.

13. A computing device for generating long-term heart rate variability based on a short-term measured heart signal, the computing device comprising:
a processor including at least one core;
memory including program codes executable on the processor; and
a network unit configured to obtain heart data including a heart signal measured within a first reference time;
wherein the processor generates heart rate variability for a second reference time, which is a value larger than the first reference time, based on the heart data by using a pre-trained deep learning model.
